# EUROPEAN PATENT SPECIFICATION

(11) **EP 2 254 642 B1**
(45) Date of publication and mention of the grant of the patent: **02.01.2019**
(21) Application number: 09722164.2
(22) Date of filing: 18.03.2009
(51) Int. Cl.: A61M 25/00, G01N 33/66

(54) **INTRAVASCULAR SENSOR AND INSERTION SET COMBINATION**
KOMBINATION AUS INTRAVASKULÄREM SENSOR UND EINFÜHRSET
DÉTECTEUR INTRAVASCULAIRE ET COMBINAISON D'ENSEMBLE D'INTRODUCTION

(30) Priority: 21.03.2008 US 52985
(43) Date of publication of application: 01.12.2010
(73) Proprietor: NOVA BIOMEDICAL CORPORATION, Waltham, MA 02454-9141 (US)
(72) Inventor: BICKOFF, Charles, Sharon Massachusetts 02067 (US); JOBST, Gerhard, 79108 Freiburg (DE)
(74) Representative: Katérle, Axel
(86) International application number: PCT/IB2009/051148
(87) International publication number: WO 2009/116000

(56) References cited:
- US-A- 4 526 432
- US-A- 6 120 515
- US-A1- 2001 025 185
- US-A1- 2002 077 680
- US-A1- 2005 183 954
- US-A1- 2006 293 576
- US-A1- 2007 178 717
- US-A1- 2007 219 441
- US-A1- 2008 064 944
- US-B1- 6 533 783

## Description

### BACKGROUND OF THE INVENTION

### 1. Field of the Invention

The present invention relates generally to the field of medical devices. Particularly, the present invention relates to devices and methods for placing a sensor at a selected site within the body of a patient. More particularly, the present invention relates to an intravascular sensor and an insertion set therefor.

### 2. Description of the Prior Art

In the past, it was discovered that tight glycemic control in critically ill patients yielded statistically beneficial results in reducing mortality of patients treated in the intensive care unit for more than five days. A study done by Greet Van den Berghe and associates (New England Journal of Medicine, November 8, 2001) showed that using insulin to control blood glucose within the range of 80- 110 mg/d L yielded statistically beneficial results in reducing mortality of patients treated in the intensive care unit for more than 5 days from 20.2 percent with conventional therapy to 10.6 percent with intensive insulin therapy. Additionally, intensive insulin control therapy reduced overall in-hospital mortality by 34 percent.

Attempts have been made in the past to monitor blood various analytes using sensors specific for the analytes being monitored. Most methods have involved reversing the direction of blood flow in an infusion line so that blood is pulled out of the patient's circulation at intervals, analyzed and then re-infused back into the patient by changing the direction of flow. A problem encountered in reversing an infusion line for sampling is determining how much blood should be withdrawn in order to be certain that pure, undiluted blood is in contact with the sensor.

U.S. Patent No. 5,165,406 (1992; Wong) discloses a sensor assembly for a combination infusion fluid delivery system and blood chemistry analysis system. The sensor assembly includes a sensor assembly with each of the assembly electrodes mounted in an electrode cavity in the assembly. The system includes provision for delivering the infusion fluid and measuring blood chemistry during reinfusion of the blood at approximately the same flow rates.

US 2005/183954 A1 describes a biosensor including a sensor shaft with a sensor cable extending therefrom, electrodes and a hub for attaching the biosensor to an introducer catheter. Further, conductive paths exist between the electrodes and first and second electrical contacts, wherein the electrical contacts are associated with the hub and a signal may be received from the first and second contacts. The biosensor may include a probe portion of which the proximal end is associated with the hub.

US 4,526,432 A relates to an electrical connector assembly for coupling flat cable conductors to electrical terminals.

Document US 2007/219441 A1 discloses a single lumen or multi-lumen intravenous catheter assembly including an integral biosensor. The catheter assembly includes one or more electrical connectors which are connected by a conduit to a junction, wherein the junction may also be connected to infusion portions via lumen. The biosensor maybe positioned within the lumen extending within a tube along a bottom portion of a catheter and, at the proximal end of the biosensor, electrical wires are coupled to or provide a conductive path through the lumen in the conduit that may terminate at the electrical connector.

US 2006/293576 A1 describes a sensor which is affixed to assembly means, wherein the assembly means are adapted to be coupled to an intravenous catheter. The sensing portion is joined to a connection portion that terminates in conductive contact pads, wherein the connection portion can be connected electrically to a monitor.

Document US 2007/178717 A1 relates to electrical connectors and also teaches a medical imaging catheter, wherein a medical positioning sensor coil is located at a distal end often imaging core included in a first assembly of the catheter.

US 2008/006 4944 A1 discloses a sensor including a sensor head having conductive contact pads thereon. The sensor is carried by an insertion set which comprises a mounting base with a tubular element defining a central bore in which the elongated sensor can be received so as to be disposed on a recessed channel of the mounting base. Further, the tubular element includes a rear cable fitting with a recessed seat sized and shape to receive and support the sensor head.

US.S. Patent No. 7,162,290 (2007; Levin) discloses a method and apparatus for periodically and automatically testing and monitoring a patient's blood glucose level. A disposable testing unit is carried by the patient's body and has a testing chamber in fluid communication with infusion lines and a catheter connected to a patient blood vessel. A reversible peristaltic pump pumps the infusion fluid forwardly into the patient blood vessel and reverses its direction to pump blood into the testing chamber to perform the glucose level test. The presence of blood in the testing chamber is sensed by a LED/photodetector pair or pairs. When the appropriate blood sample is present in the test chamber, a glucose oxidase electrode is energized to obtain the blood glucose level.

Although Levin discloses a method of halting the withdrawal of blood at the proper time so that a pure, undiluted sample is presented to the sensor, the method uses an expensive sensor and risks the possibility of contamination by the infusion process. Additionally, infusion of the flush solution has a diluting effect of the blood in the vicinity of the intravenous catheter and presents a time dependent function as to the frequency at which blood glucose can be measured.

Therefore, what is needed is a device that simplifies the measurement apparatus. What is also needed is a device that improves usability and limits the infusion fluid to the level required to clear the intravenous catheter site. What is further needed is a device that simplifies the procedures required of medical personnel to those closely related to existing accepted methods.

### SUMMARY OF THE INVENTION

It is an object of the present invention to provide a device that simplifies the components needed for the measurement apparatus. It is another object of the present invention to provide a device that improves usability and simplifies the procedures to those closely related to existing accepted method known to medical personnel.

The present invention achieves these and other objectives by providing an intravascular sensor and insertion set combination according to independent claims 1 and 7. A sensor assembly is configured for use with commercially available intravenous insertion devices. The sensor assembly includes a sensor sheath having a diameter substantially similar to a commercially available catheter insertion needle so that the sensor sheath sealingly
engages the distal end of the catheter when the sensor assembly is inserted into the catheter after removal of the insertion needle.

The sensor sheath contains a sensor having sensing elements disposed on a sensor shank adjacent a sensor distal end and electrical contacts at or adjacent a sensor proximal end. The sensor shank is sealingly embedded within the sensor sheath where the sensor elements are exposed at or adjacent the sensor distal end. The sensor sheath includes a hub configured for mating with the luer lock fitting on the catheter. A secondary seal is made at the luer fitting. The sensor may include one or more sensing elements on one side or on opposite sides of the sensor shank.

The sensor signals are transmitted to a monitor by cabling or by radio waves. An optional signal conditioning electronics may be included to receive the sensor signals by way of electrical leads from the sensor. Either hard wiring or a radio link communicates the sensor signals to a monitor, which processes the sensor signals and displays analytical values, trends and other patient related data for the measured analyte. A typical analyte is blood glucose. Blood glucose measurements are commonly used to determine insulin dosing in tight glycemic control protocols. Although blood glucose is an important blood component, other analytes are possible to measure within the constructs of the present invention.

One of the major advantages of the present invention is the combination with commercially-available IV catheters. This simplifies the procedure required of medical personnel since no additional special techniques are required for inserting the intravenous catheter. No highly specialized training is required since the procedures used by medical personnel to insert the intravascular sensor are closely related to existing accepted methods. Upon removal of the insertion needle, the sensor assembly of the present invention is simply inserted and locked into place using the luer lock fitting. Because the present invention is configured for use with commercially-available IV catheters, no specially designed or customized insertion tools or devices are required to position the intravascular sensor in the patient.

### BRIEF DESCRIPTION OF THE DRAWINGS

FIGURE 1 is a plan view showing the general installation of the intravenous catheter and sensor on a patient in a direct connection to the monitor.
FIGURE 2 is a plan view showing the general installation of the intravenous catheter and sensor on a patient in a radio communication connection to the monitor.
FIGURE 3 is a perspective view of one embodiment of the present invention showing the intravascular sensor insertion set.
FIGURE 4 is an exploded view of the assembled sensor and cable of the present invention shown in Fig. 3.
FIGURE 5 is an end view of the cable end of the hub of the present invention showing the cross section of the sensor sheath.
FIGURE 6 is a perspective view of one embodiment of the sensor of the present invention showing contact wings.
FIGURE 7 is an enlarged perspective view of the contact wings shown in Fig. 6.
FIGURE 8 is an enlarged perspective view showing the sensor element end in one embodiment of the sensor.
FIGURE 9 is an enlarged end view of the hub of the present invention showing the connection between the cable and the connector end of the sensor.
FIGURE 10 is a perspective view of one embodiment of the present invention showing the sensor assembly inserted into the intravenous catheter.
FIGURE 11 is a cross-sectional view of the sensor inserted into the intravenous catheter.
FIGURE 12 is an enlarged perspective view of one embodiment of the present invention showing the sheath with a side opening/window exposing the sensor elements.
FIGURE 13 is an enlarged perspective view of another embodiment of the present invention showing the sensor and sheath end with the intravenous catheter where all sensor elements are on one side.
FIGURE 14 is an enlarged cross-sectional view of the embodiment of the sensor assembly and intravenous catheter shown in Fig. 13.
FIGURE 15 is a perspective view of another embodiment of the present invention showing the sensor assembly inserted into the intravenous catheter where the sensor elements extend beyond the end of the sensor sheath.
FIGURE 16 is an enlarged perspective view of the sensor elements shown in Fig. 15.

### DETAILED DESCRIPTION OF THE PREFERRED EMBODIMENT

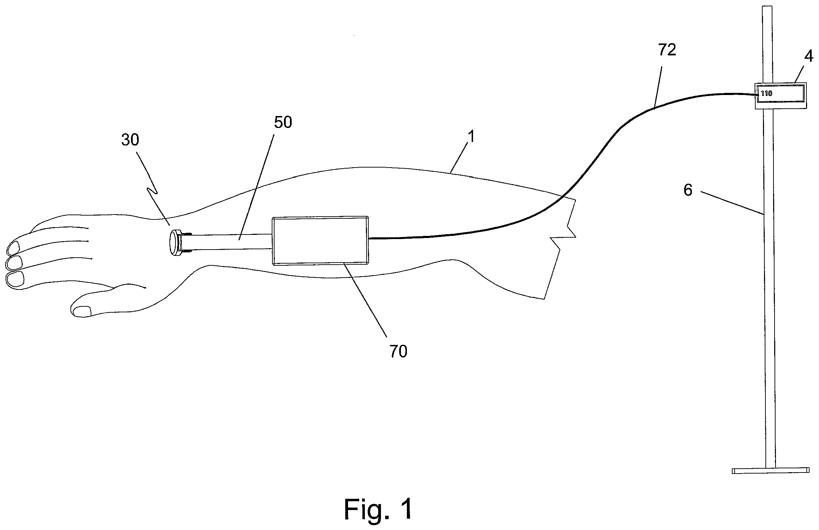

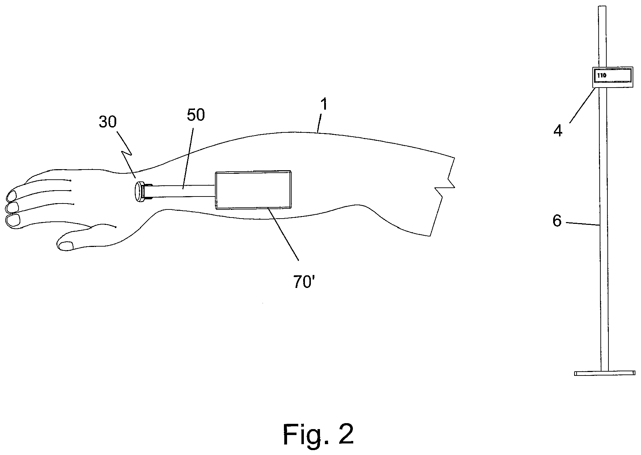

The preferred embodiment(s) of the present invention is illustrated in Figs. 1-16. Figures 1 and 2 illustrate the overall environment of the present invention connected to an arm 1 of a patient. Fig. 1 shows, by way of example, a disposable sensor assembly 30 of the present invention inserted into the intravascular system of the patient, which has been inserted into a vein on the back of arm 1 above the wrist. A catheter assembly 20 (not shown) is preferably used with the present invention and together with the sensor assembly 30 make up one embodiment of the intravascular sensor insertion set 10 of the present invention. Additionally, other locational installations on the patient are possible and often used.

As shown in Figure 1, a sensor cable 50 emanates from the sensor assembly 30 and is attached to a conditioning electronics and a cable junction unit 70. A monitor cable 72 electrically couples cable junction unit 70 to a monitor 4 mounted on a pole 6. Such poles as pole 6 are often used to mount electronic equipment as well as intravenous drips and the like. Another common location for the monitor 4 is the bed rail. Monitor cable 72 and sensor cable 50 transmit electrical signals generated by the sensor assembly 30 directly to monitor 4 where the signals are processed and displayed for access by medical personnel. Cable junction unit 70 is shown for convenience, as it is possible for monitor cable 72 and sensor cable 50 to be a single entity. It should be noted that other mounting configurations other than mounting monitor 4 to pole 6 is possible. For instance, it is possible to mount monitor 4 to a bed rail, cart mount, or other convenient location and often desirable.

Like the illustration in Fig. 1, Figure 2 shows a sensor cable 50 emanating from the sensor assembly 30 and attached to a conditioning electronics and radio unit 70'. The conditioning electronics and radio unit 70' transmits electrical signals generated by the sensor assembly 30 to the monitor 4 where the signals are processed and displayed for access by medical personnel.

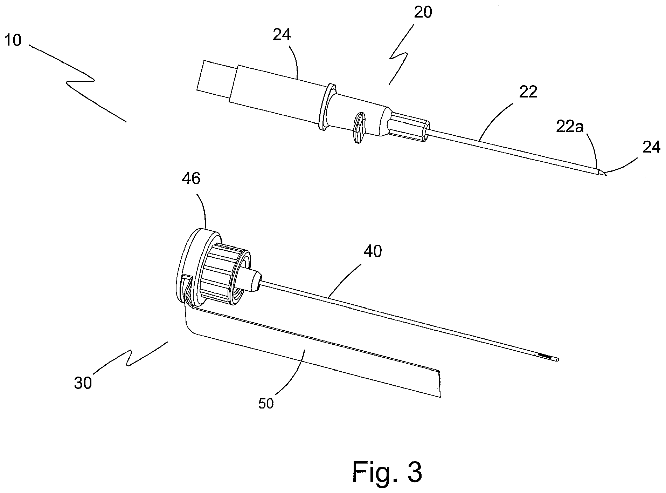

Turning now to Figure 3, there is illustrated one embodiment of the intravascular sensor insertion set 10 of the present invention. Sensor insertion set 10 includes sensor assembly 30 and catheter assembly 20. Sensor assembly 30 includes a sensor sheath 40 sealingly connected to a sensor hub 46 from which sensor cable 50 extends. Catheter assembly 20 typically includes an insertion needle 24 disposed within a flexible catheter 22 and extends a predefined distance beyond a catheter distal end 22a. Sensor assembly 30 is preferably constructed to be insertable into a commercially available intravenous catheter assembly 20 that are typically available from a variety of medical suppliers. Some examples of these commercially available intravenous catheter assemblies include intravenous insertion catheters sold under the trademarks Introcan (manufactured by B. Braun) and Insyte Autoguard (manufactured by Becton Dickinson).

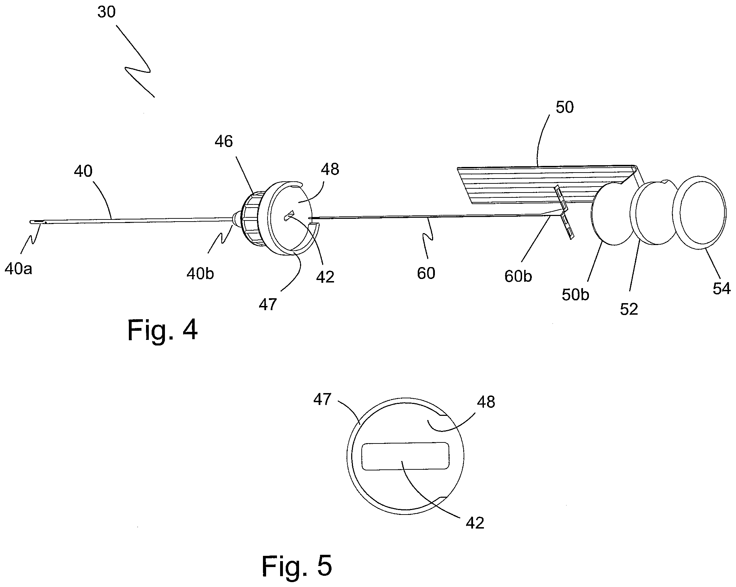

Figure 4 is an exploded view of sensor assembly 30 shown in Fig. 3. Sensor assembly 30 includes sensor sheath 40, sheath hub 46, a sensor 60, and sensor cable 50. Sensor sheath 40 includes a sheath distal end 40a and a sheath proximal end 40b. Sheath proximal end 40b is sealingly affixed to sheath hub 46. Sensor sheath 40 includes an internal channel 42 that extends substantially the entire length of sheath 40 and receives sensor 60. Sensor 60 has a shank proximal end 60b that is received within hub 46 against a hub surface 48 along with a sensor cable proximal end 50b. Sensor 60 and cable proximal end 50b are fixedly retained within hub 46 by a pressure applying component 52 and a pressure cap 54. Pressure applying component 52 is optionally made from a resilient material such as a foam material that is placed over cable proximal end 50b to apply pressure between cable proximal end 50b and shank proximal end 60b. Pressure cap 54 provides the mechanism for maintaining the applied pressure and is preferably permanently affixed to hub 46.

Figure 5 is an enlarged plan view of hub surface 48. Internal channel 42 has a cross-section that is suitable for receiving sensor 60 and can be any desired shape. Hub 46 optionally has a perimeter wall 47 around a major portion of the circumference of hub surface 48. Perimeter wall 47 facilitates attaching pressure cap 54 when capturing sensor 60, cable proximal end 50b and pressure applying component 52. Pressure cap 54 may be fixed to hub 46 by a snap fit, ultrasonic welding, chemical welding, or the like. Although cable 50 is shown as a flex circuit, it should be understood that other cable topologies are possible.

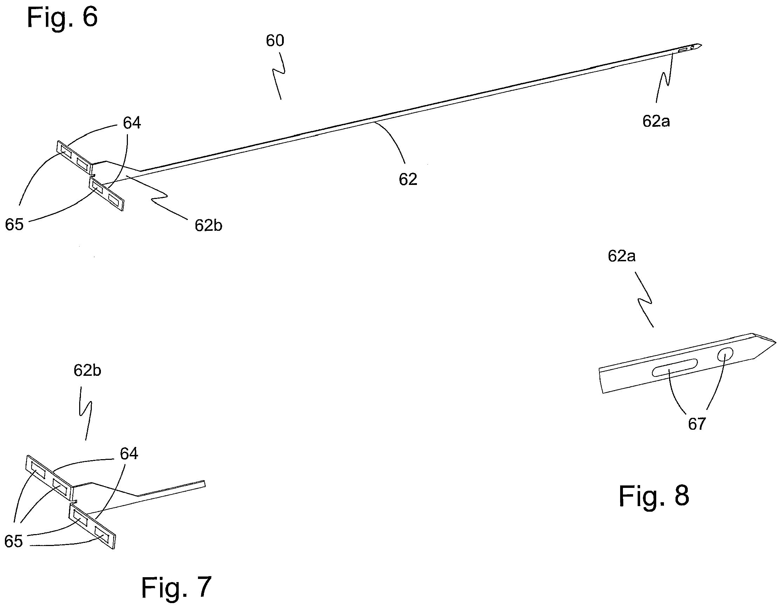

Figure 6 shows one embodiment of sensor 60 of the present invention. Sensor 60 has a sensor shank 62 with a shank distal end 62a and shank proximal end 62b. Shank proximal end 62b has contact ears 64 that have been orthogonally folded outward from sensor shank 62. Contact ears 64 carry electrical contact pads thereon, which are more clearly illustrated in Figure 7. Turning now to Fig. 7 there is illustrated an enlarged view of shank proximal end 62b. Contact ears 64 have exposed thereon a plurality of electrical contact pads 65. By configuring contact ears 64 as shown, electrical contact pads 65 are all facing in one direction facilitating connection to a single-sided sensor cable 50 such as a flex cable. Figure 8 is an enlarged view of shank distal end 62a. Shank distal end 62a has one or more sensor elements 67. Each of the one or more sensor elements 67 are electrically coupled to contact pads 65, typically by embedding one or more electrically conductive pathways (not shown) within sensor shank 62 where the electrically conductive pathways are electrically isolated from each other. In this particular embodiment, sensor elements 67 of sensor 60 are on both sides. Other quantities of electrical contacts and sensor elements are considered within the scope of the present invention.

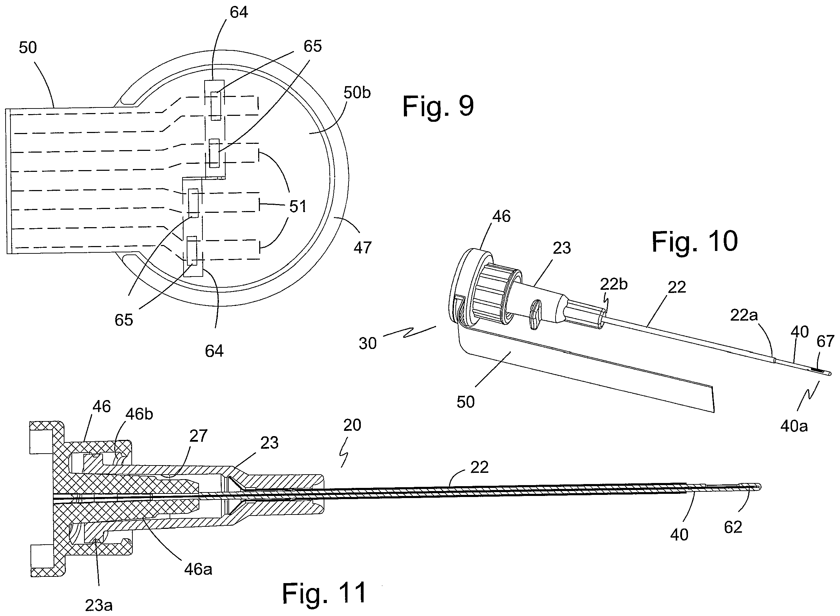

Turning now to Figure 9, there is illustrated an enlarged plan view of the electrical coupling assembly within hub 46. Cable 50 has a plurality of electrical conductors 51 that terminate at cable proximal end 50b. A portion of electrical conductors 51 are exposed and overlay against electrical contacts 65 of contact ears 64. As shown, cable proximal end 50b is preferably shaped to be captured within perimeter wall 47 of hub 46. As previously disclosed, pressure applying component 52 (not shown) is positioned on top of cable proximal end 50b. In this embodiment, pressure applying component 52 has a thickness greater than the height of perimeter wall 47 so that pressure cap 54, when installed, pushes pressure applying component 53 against cable proximal end 50b in order to maintain good electrical contact between electrical contacts 65 of contact ears 64 and the corresponding portions of exposed electrical conductors 51 at cable proximal end 50b.

Sensor assembly 30 positioned within catheter 22 is illustrated in Figure 10. Catheter 22 includes a luer fitting 23 attached permanently and hermetically to a catheter proximal end 22b to form a leak-proof entity. A catheter distal end 22a is tapered so that a liquid tight seal is formed between the inside diameter of catheter 22 and insertion needle 24 (not shown). The diameter of sensor sheath 40 is selected to be substantially the same as the diameter of insertion needle 24 so that, when sensor assembly 30 is inserted into catheter 22 after removal of insertion needle 24, a liquid tight seal is also formed at catheter distal end 22a between catheter distal end 22a and sensor sheath 40. As Fig. 10 illustrates, a sheath distal end 40a containing sensing elements 67 extends beyond catheter distal end 22a in order to expose sensing elements 67 to the sample fluid, i.e. the blood within the vein of the patient.

Luer fitting 23 removably connects to hub 46 of sensor assembly 30 in a similar fashion as the standard luer-lock connections known to those of ordinary skill in the art. Figure 11 is a cross sectional view which particularly shows the luer lock interface between the luer taper 46a of the sheath hub 46 and the luer taper 27 of the luer lock fitting 23 of the intravenous catheter assembly 20. The threads 23a of the luer lock fitting 23 of the intravenous catheter assembly 20 threadingly engages with the threads 46b of the sheath hub 46.

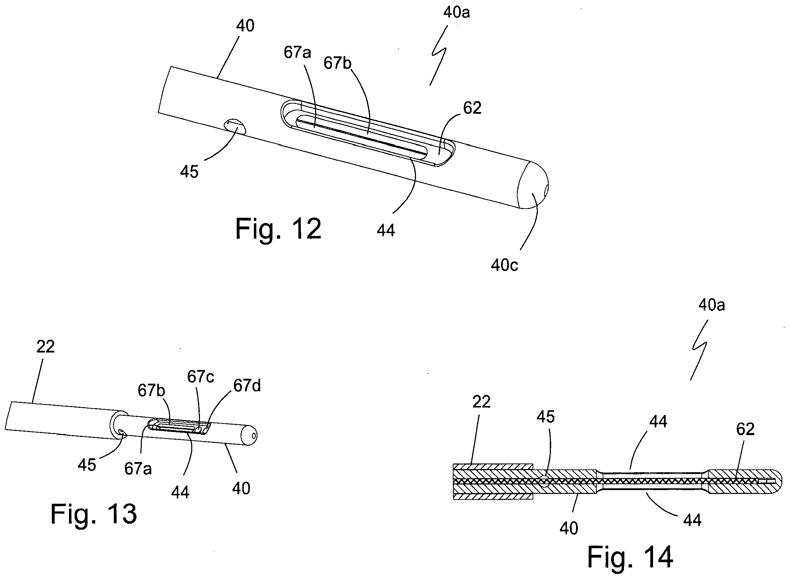

Turning now to Figure 12 there is illustrated an enlarged perspective view of one embodiment of the sensor elements 67 of sensor 60. Sensor sheath 40 has a side opening 44, i.e. window, near sheath distal end 40a. Two sensor elements 67a, 67b on sensor shank 62 are disposed at side opening 44. In this embodiment, sheath distal end 40a has a sealed end 40c. Sensor sheath 40 also includes a cross-drilled opening 45 to provide access for disposing a sealant around sensor shank 62 and sheath channel 42 at sheath distal end 40a to form a liquid tight seal. It should be noted that sensor sheath 40 may optionally include additional side openings or windows to accommodate additional sensor elements to measure a plurality of blood analytes.

Figure 13 shows another embodiment of sensor assembly 30 where all sensor elements 67a, 67b, 67c, and 67d are on the same side of sensor shank 62. Sensor elements 67a, 67b, 67c, and 67d are positioned with sheath 40 to be located beneath sheath side opening 44. Sheath 40 also includes cross-drilled opening 45 for applying sealant around sensor shank 62 and sheath channel 42 to form a liquid tight seal. Figure 14 is a cross-section view of the embodiment in Figure 13. Figure 14 more clearly shows the relational detail of sensor shank 62, sheath side opening 44 and cross-drilled opening 45.

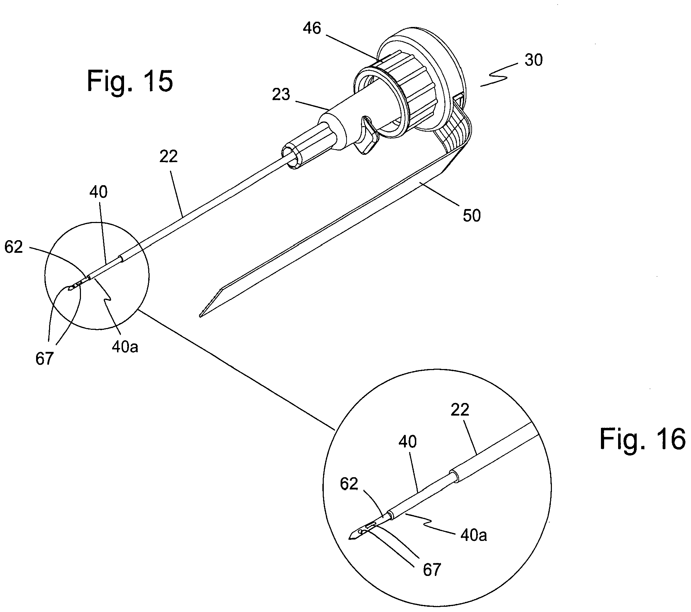

Figure 15 is a perspective view of another embodiment of the present invention. In this combination of sensor assembly 30 and catheter 40, sensor elements 67 are not protectively disposed beneath a window in sensor sheath 40 but positioned on a portion of sensor shank 62 that extends beyond sheath distal end 40a. Figure 16 is an enlarged detail view of the distal end of the embodiment in Fig. 15. Fig. 16 more clearly shows the relational detail between sensor elements 67, sensor shank 62, sensor sheath 40, and catheter 22.

Because sensor 60 is positioned within sensor sheath 40, sensor shank 62 may have a characteristic of being rigid or flexible or any degree of rigidity/flexibility. Preferably, sensor shank 62 is flexibly resilient to provide less susceptibility to damage during handling and use when configured for any embodiment of the present invention. The following is one example for fabricating a sensor 60 of the present invention.

### Sensor Fabrication

Step 1. Obtain a sheet of polyimide film, preferably with a thickness of about 0.002 inches. One option to obtain such a polyimide film is to remove the copper layer from a sheet of polyimide flexible laminate available from E. I. du Pont de Nemours and Company, Cat. No. AP8525 under the trademark Pyralux ®. Pyralux® AP double-sided, copper-clad laminate is an all-polyimide composite polyimide film bonded to copper foil. Chemical etching is the preferred method for removing the copper layer. The polyimide sheet will become the polyimide support substrate for the sensor elements 67 of the present invention.
Step 2. Apply liquid photoresist to both sides of the polyimide support substrate, expose the photoresist to UV light in a predefined pattern, and remove the unexposed areas to create a pattern for metal deposition. It should be understood that the preferred embodiment of the present invention has sensor elements 67 on both sides of the support substrate but that a single-sided sensor can also be made and is within the scope of the present invention. It is also understood that isolated electrically-conductive pathways are defined in the pattern between each sensor element 67 and a corresponding electrical contact 65. A single sheet of polyimide support substrate provides a plurality of sensors 60. Typically, one side contains the defined two electrodes per sensor (referred to as the top side) while the opposite side contains the reference and/or counter electrodes (referred to as the backside).
Step 3. Coat both sides with one or more layers of electrically conductive materials by vacuum deposition. Acceptable electrically conductive materials include platinum, gold, and the like. Preferably, platinum with a layer of titanium deposited thereon is used for the present invention.
Step 4. Remove the photoresist including the electrically conductive material on top of the photoresist surface leaving a pattern of electrically conductive material on the polyimide surfaces.
Step 5. Apply an insulation layer to both sides of the modified polyimide sheet preferably by lamination. The insulation layer is preferably a flexible photoimageable coverlay available from E. I. du Pont de Nemours and Company as Pyralux® PC. Pyralux® PC is a flexible, dry film solder mask used to encapsulate flexible printed circuitry. The dry film can be used as a solder mask by patterning openings using conventional printed circuit exposure and development processes. Unexposed areas can be developed off as explained in the technical information brochure provided by Dupont. For the present invention, Pyralux® PC 1015 was used. Expose the insulation layer to UV light and wash out the unexposed portions of the insulation layer. Thermally cure the remaining insulation layer/dry film. The cured remaining insulation layer serves as not only an insulation layer but also forms the wells to confine and contain the dispensed layers disclosed below.
Step 6. Remove the titanium in the areas exposed by the insulation layer using aqueous hydrofluoric acid, which also conveniently removes any surface contaminants from the previous process.
Step 7. Deposit silver onto the electrodes defined by the electrically conductive material pattern on the backside of the polyimide support substrate, and subsequently convert a portion to silver chloride to create a Ag/AgCl electrode, which will serve as counter and reference electrode.
Step 8. Deposit a semi-permeable membrane to the two electrodes per sensor defined on the top side (i.e. glucose electrode and blank electrode) by electropolymerization.
Step 9. Deposit a hydrogel membrane onto the Ag/AgCl counter and reference electrode on the backside of the sheet by dispensing a predefined amount of hydrogel membrane solution, followed by UV curing and washing.
Step 10. Deposit a poly-2-hydroxyethyl methacrylate (PHEMA) membrane precursor solution onto the two electrodes per sensor defined on the top side, UV cure, wash and dry. It should be understood by those skilled in the art that one of the two electrodes is a glucose electrode and, accordingly, the PHEMA membrane precursor solution for this electrode additionally contains a glucose enzyme, preferably glucose oxidase.
Step 11. Deposit a composite membrane precursor solution onto the glucose electrode and the blank electrode, UV cure and dry. The preparation of the composite membrane precursor solution will now be described. Microspheres are prepared from a material having substantially no or little permeability to glucose but a substantially high permeability to oxygen. The microspheres are preferably prepared from PDMS (polydimethylsiloxane). The microspheres are mixed with a hydrogel precursor that allows the passage of glucose. While polyurethane hydrogels work, a PHEMA precursor is preferred. The ratio of microspheres to hydrogel determines the ratio of the glucose to oxygen permeability. Thus, one of ordinary skill in the art can easily determine the ratio that enables the desired dynamic range of glucose measurement at the required low oxygen consumptions. It should be noted that if a polyurethane hydrogel is used, the membrane is cured by evaporating the solvent instead of using ultraviolet light.
Step 12. Optionally deposit additional PHEMA membrane precursor solution to the glucose and blank electrode, UV cure and dry. This optional step adds catalase that prevents release of hydrogen peroxide to the biological environment, reduces flow rate influence on sensor sensitivity and prevents direct contact of the microspheres surface to the biological environment.
Step 13. Cut the polyimide sheet into individual sensors 60.

The individual sensors 60 are then assembled into the sensor sheath 40 according to the preferred embodiments previously described.

Although the preferred embodiments of the present invention have been described herein, the above description is merely illustrative. Further modification of the invention herein disclosed will occur to those skilled in the respective arts and all such modifications are deemed to be within the scope of the invention as defined by the appended claims.

## Claims

1. An intravascular sensor assembly (30) insertable into an intravenous catheter, the sensor assembly comprising:
a sheath (40) having a sheath proximal end (40b) and a sheath distal end (40a) wherein an outer diameter of the sheath (40) is sized to be substantially equal to the outer diameter of an insertion needle of the intravenous catheter;
a hub (46) sealingly connected to the sheath proximal end (40b), the hub (46) adapted for removably coupling to the intravenous catheter;
a sensor (60) disposed within the sheath (40), the sensor (60) having a sensor shank (62) with a shank distal end (62a) and a shank proximal end (62b), a plurality of sensor elements (67) including a working electrode (67a) with a reagent matrix disposed thereon, the reagent matrix comprising an enzyme capable of catalyzing a reaction involving a substrate for the enzyme and a reference electrode (67b), the plurality of sensor elements (67) disposed adjacent the shank distal end (62a), a plurality of connector pads (65) disposed at the shank proximal end (62b) wherein the plurality of connector pads (65) are contained within the hub (46) and the plurality of sensor elements (67) are exposed adjacent the sheath distal end (40a), and a plurality of elongated conductive elements (51) wherein each of the plurality of conductive elements (51) electrically couples one of the plurality of sensor elements (67) to one of the plurality of connector pads (65); and
a cable (50) electrically coupled to the plurality of connector pads (65), **characterized in that**:
the shank proximal end (62b) of the sensor shank (62) has a pair of contact ears (64) orthogonally folded outward in opposite directions from the sensor shank (62) so that the contact ears (64) are disposed substantially perpendicular to the longitudinal axis of the sensor shank (62), the contact ears (64) having the connector pads (65) exposed thereon, the connector pads (65) all facing in one direction; and
the contact ears (64) are seated against a reference surface (48) within the hub (46) at a base of the hub (46) adjacent a sheath-hub interface.

2. The sensor of Claim 1 wherein the plurality of sensor elements (67) and the shank distal end (62a) are exposed beyond the sheath distal end (40a).

3. The sensor of Claim 1 wherein the plurality of sensor elements (67) is exposed at an opening (44) in the sheath (40) adjacent the sheath distal end (40a).

4. The sensor of Claim 1 wherein the plurality of sensor elements (67) is exposed at orthogonal openings (44) on opposite sides of the sheath adjacent the sheath distal end (40a).

5. The sensor of Claims 1 to 4 wherein the hub (46) further includes a resilient component (52) disposed within the hub (46) against the cable (50b) and a pressure cap (54) fixedly attached to the hub (46) and sized to provide pressure on the resilient component (52) causing the cable (50) and the connector pads (65) to remain in intimate electrical contact.

6. The sensor of Claims 1 to 5 further includes conditioning electronics (70) coupled to the cable (50), the conditioning electronics (70) communicatingly coupled to a monitor (4).

7. In combination, an intravascular sensor assembly (30) and an intravenous catheter assembly (20), the combination comprising:
an intravenous catheter assembly (20) comprising an intravenous catheter (22) and an intravenous insertion needle (24) removably and slidably disposed within the intravenous catheter (20); and
an intravascular sensor assembly (30) comprising a sheath (40), a hub (46) sealingly connected to a proximal end (40b) of the sheath (40), and a sensor (60) sealingly disposed within the sheath (40) wherein the sensor (60) has a sensor shank (62) with a plurality of sensor elements (67) including a working electrode (67a) with a reagent matrix disposed thereon, the reagent matrix comprising an enzyme capable of catalyzing a reaction involving a substrate for the enzyme and a reference electrode (67b), the plurality of sensor elements (67) disposed adjacent a shank distal end (62a), a plurality of connector pads (65) disposed at a shank proximal end (62a) wherein the plurality of connector pads (65) are contained within the hub (46) and the plurality of sensor elements (67) are exposed adjacent a sheath distal end (40a), and a plurality of elongated conductive elements (51) wherein each of the plurality of conductive elements (51) electrically couples one of the plurality of sensor elements (67) to one of the plurality of connector pads (65), and a cable electrically (50) coupled to the plurality of connector pads (65);
wherein the intravascular sensor assembly (30) is removably and sealingly insertable into the intravenous catheter (22) after removal of the insertion needle (24),
**characterized in that**:
the shank proximal end (62b) of the sensor shank (62) has a pair of contact ears (64) orthogonally folded outward in opposite directions from the sensor shank so that the contact ears are disposed substantially perpendicular to the longitudinal axis of the sensor shank, the contact ears having the connector pads (65) exposed thereon, the connector pads all facing in one direction; and
the contact ears (64) are seated against a reference surface (48) within the hub (46) at a base of the hub adjacent a sheath-hub interface.

8. The combination of Claim 7 wherein the plurality of sensor elements (67) and the shank distal end (62) are exposed beyond the sheath distal end (40a).

9. The combination of Claim 7 wherein the plurality of sensor elements (67) is exposed at an opening (44) in the sheath adjacent the sheath distal end (40a).

10. The combination of Claim 7 wherein the plurality of sensor elements (67) is exposed at orthogonal openings (44) on opposite sides of the sheath adjacent the sheath distal end (40a).

11. The combination of Claims 7 to 10 wherein the hub (46) further includes a resilient component (52) disposed within the hub (46) against the cable (50) and a pressure cap (54) fixedly attached to the hub (46) and sized to provide pressure on the resilient component (52) causing the cable (50) and the connector pads (65) to remain in intimate electrical contact.

## Patentansprüche

1. Intravaskuläre Sensoranordnung (30), die in einen intravenösen Katheter einsetzbar ist, wobei die Sensoranordnung umfasst:
eine Hülse (40) mit einem proximalen Hülsenende (40b) und einem distalen Hülsenende (40a), wobei ein Außendurchmesser der Hülse (40) so bemessen ist, dass er im Wesentlichen gleich dem Außendurchmesser einer Einführnadel des intravenösen Katheters ist;
eine Nabe (46), die abdichtend mit dem proximalen Hülsenende (40b) verbunden ist, wobei die Nabe (46) zum lösbaren Verbinden mit dem intravenösen Katheter angepasst ist;
einen Sensor (60), der innerhalb der Hülse (40) angeordnet ist, wobei der Sensor (60) einen Sensorschaft (62) mit einem distalen Schaftende (62a) und einem proximalen Schaftende (62b), eine Mehrzahl von Sensorelementen (67) einschließlich einer Arbeitselektrode (67a) mit einer darauf angeordneten Reagenzmatrix, wobei die Reagenzmatrix ein Enzym umfasst, das eine Reaktion katalysieren kann, die ein Substrat für das Enzym umfasst, und eine Referenzelektrode (67b), wobei die Mehrzahl von Sensorelementen (67) angrenzend an das distale Schaftende (62a) angeordnet ist, eine Mehrzahl von Anschlussflächen (65), die an dem proximalen Schaftende (62b) angeordnet sind, wobei die Mehrzahl von Anschlussflächen (65) innerhalb der Nabe (46) enthalten ist und die Mehrzahl von Sensorelementen (67) angrenzend an das distale Hülsenende (40a) freigelegt ist, und eine Mehrzahl von langgestreckten leitfähigen Elementen (51) aufweist, wobei jedes der Mehrzahl von leitfähigen Elementen (51) eines der Mehrzahl von Sensorelementen (67) elektrisch mit einer der Mehrzahl von Anschlussflächen (65) verbindet; und
ein Kabel (50), das elektrisch mit der Mehrzahl von Anschlussflächen (65) verbunden ist, **dadurch gekennzeichnet, dass**:
das proximale Schaftende (62b) des Sensorschafts (62) ein Paar von Kontaktohren (64) aufweist, die orthogonal in entgegengesetzten Richtungen von dem Sensorschaft (62) nach außen gefaltet sind, so dass die Kontaktohren (64) im Wesentlichen senkrecht zu der Längsachse des Sensorschafts (62) angeordnet sind, wobei die Kontaktohren (64) darauf die freigelegten Anschlussflächen (65) aufweisen und wobei die Anschlussflächen (65) alle in eine Richtung weisen; und
die Kontaktohren (64) an einer Referenzoberfläche (48) innerhalb der Nabe (46) an einer Basis der Nabe (46) angrenzend an eine Hülse-Nabe-Schnittstelle sitzen.

2. Sensor nach Anspruch 1, wobei die Mehrzahl von Sensorelementen (67) und das distale Schaftende (62a) über das distale Hülsenende (40a) hinaus freigelegt sind.

3. Sensor nach Anspruch 1, wobei die Mehrzahl von Sensorelementen (67) an einer Öffnung (44) in der Hülse (40) angrenzend an das distale Hülsenende (40a) freigelegt ist.

4. Sensor nach Anspruch 1, wobei die Mehrzahl von Sensorelementen (67) an orthogonalen Öffnungen (44) auf gegenüberliegenden Seiten der Hülse angrenzend an das distale Hülsenende (40a) freigelegt ist.

5. Sensor nach einem der Ansprüche 1 bis 4, wobei die Nabe (46) ferner ein elastisches Element (52), das innerhalb der Nabe (46) an dem Kabel (50b) angeordnet ist, und eine Druckkappe (54) umfasst, die fest an der Nabe (46) angebracht ist und so bemessen ist, dass ein Druck auf das elastische Element (52) ausgeübt wird, der bewirkt, dass das Kabel (50) und die Anschlussflächen (65) in engem elektrischen Kontakt bleiben.

6. Sensor nach einem der Ansprüche 1 bis 5, ferner umfassend eine mit dem Kabel (50) verbundene Bearbeitungselektronik (70), wobei die Bearbeitungselektronik (70) kommunizierend mit einem Monitor (4) verbunden ist.

7. Kombination aus einer intravaskulären Sensoranordnung (30) und einer intravenösen Katheteranordnung (20), wobei die Kombination umfasst:
eine intravenöse Katheteranordnung (20), die einen intravenösen Katheter (22) und eine intravenöse Einführnadel (24) umfasst, die entfernbar und verschiebbar innerhalb des intravenösen Katheters (20) angeordnet ist; und
eine intravaskuläre Sensoranordnung (30), umfassend eine Hülse (40), eine Nabe (46), die abdichtend mit einem proximalen Ende (40b) der Hülse (40) verbunden ist, einen Sensor (60), der abdichtend innerhalb der Hülse (40) angeordnet ist, wobei der Sensor (60) einen Sensorschaft (62) mit einer Mehrzahl von Sensorelementen (67) einschließlich einer Arbeitselektrode (67a) mit einer darauf angeordneten Reagenzmatrix, wobei die Reagenzmatrix ein Enzym umfasst, das eine Reaktion katalysieren kann, die ein Substrat für das Enzym umfasst, und eine Referenzelektrode (67b), wobei die Mehrzahl von Sensorelementen (67) angrenzend an ein distales Schaftende (62a) angeordnet ist, eine Mehrzahl von Anschlussflächen (65), die an einem proximalen Schaftende (62a) angeordnet sind, wobei die Mehrzahl von Anschlussflächen (65) innerhalb der Nabe (46) enthalten ist und die Mehrzahl von Sensorelementen (67) angrenzend an ein distales Hülsenende (40a) freigelegt ist, und eine Mehrzahl von langgestreckten leitfähigen Elementen (51) aufweist, wobei jedes der Mehrzahl von leitfähigen Elementen (51) elektrisch eines der Mehrzahl von Sensorelementen (67) mit einer der Mehrzahl von Anschlussflächen (67) verbindet, und ein Kabel (50), das mit der Mehrzahl von Anschlussflächen (65) elektrisch verbunden ist;
wobei die intravaskuläre Sensoranordnung (30) nach Entfernung der Einführnadel (24) lösbar und abdichtend in den intravenösen Katheter (22) einsetzbar ist,
**dadurch gekennzeichnet, dass**:
das proximale Schaftende (62b) des Sensorschafts (62) ein Paar von Kontaktohren (64) aufweist, die orthogonal in entgegengesetzten Richtungen von dem Sensorschaft nach außen gefaltet sind, so dass die Kontaktohren im Wesentlichen senkrecht zu der Längsachse des Sensorschafts (62) angeordnet sind, wobei die Kontaktohren darauf die freigelegten Anschlussflächen (65) aufweisen und wobei die Anschlussflächen alle in eine Richtung weisen; und
die Kontaktohren (64) an einer Referenzoberfläche (48) innerhalb der Nabe (46) an einer Basis der Nabe angrenzend an eine Hülse-Nabe-Schnittstelle sitzen.

8. Kombination nach Anspruch 7, wobei die Mehrzahl von Sensorelementen (67) und das distale Schaftende (62a) über das distale Hülsenende (40a) hinaus freigelegt sind.

9. Kombination nach Anspruch 7, wobei die Mehrzahl von Sensorelementen (67) an einer Öffnung (44) in der Hülse angrenzend an das distale Hülsenende (40a) freigelegt ist.

10. Kombination nach Anspruch 7, wobei die Mehrzahl von Sensorelementen (67) an orthogonalen Öffnungen (44) auf gegenüberliegenden Seiten der Hülse angrenzend an das distale Hülsenende (40a) freigelegt ist.

11. Kombination nach einem der Ansprüche 7 bis 10, wobei die Nabe (46) ferner ein elastisches Element (52), das innerhalb der Nabe (46) an dem Kabel (50b) angeordnet ist, und eine Druckkappe (54) umfasst, die fest an der Nabe (46) angebracht ist und so bemessen ist, dass ein Druck auf das elastische Element (52) ausgeübt wird, der bewirkt, dass das Kabel (50) und die Anschlussflächen (65) in engem elektrischen Kontakt bleiben.

## Revendications

1. Ensemble (30) de capteur intravasculaire insérable dans un cathéter intraveineux, l'ensemble de capteur comprenant :
une gaine (40) ayant une extrémité proximale (40b) de gaine et une extrémité distale (40a) de gaine dans lequel un diamètre extérieur de la gaine (40) est dimensionné pour être sensiblement égal au diamètre extérieur d'une aiguille d'insertion du cathéter intraveineux ;
un raccord (46) connecté de façon étanche à l'extrémité proximale (40b) de gaine, le raccord (46) adapté à un couplage amovible au cathéter intraveineux ;
un capteur (60) disposé à l'intérieur de la gaine (40), le capteur (60) ayant une tige (62) de capteur avec une extrémité distale (62a) de tige et extrémité proximale (62b) de tige, une pluralité d'éléments (67) de capteur incluant une électrode de travail (67a) avec une matrice de réactif disposée sur celle-ci, la matrice de réactif comprenant une enzyme apte à catalyser une réaction impliquant un substrat pour l'enzyme et une électrode de référence (67b), la pluralité d'éléments (67) de capteur disposés adjacents à l'extrémité distale (62a) de tige, une pluralité de plots (65) de connecteur disposés à l'extrémité proximale (62b) de tige dans lequel la pluralité de plots (65) de connecteur sont contenus à l'intérieur du raccord (46) et la pluralité d'éléments (67) de capteur sont exposés adjacents à l'extrémité distale (40a) de gaine, et une pluralité d'éléments conducteurs (51) allongés dans lequel chacun de la pluralité d'éléments conducteurs (51) couple électriquement un de la pluralité d'éléments (67) de capteur à un de la pluralité de plots (65) de connecteur ; et
un câble (50) électriquement couplé à la pluralité de plots (65) de connecteur,
**caractérisé en ce que** :
l'extrémité proximale (62b) de tige de la tige (62) de capteur a une paire de pattes de contact (64) repliées orthogonalement vers l'extérieur dans des directions opposées depuis la tige (62) de capteur de telle manière que les pattes de contact (64) sont disposées sensiblement perpendiculaires à l'axe longitudinal de la tige (62) de capteur, les pattes de contact (64) ayant les plots (65) de connecteur exposés sur celles-ci, les plots (65) de connecteur faisant tous face dans une direction ; et
les pattes de contact (64) sont logées contre une surface de référence (48) à l'intérieur du raccord (46) au niveau d'une base du raccord (46) adjacente à une interface gaine-raccord.

2. Capteur selon la revendication 1, dans lequel la pluralité d'éléments (67) de capteur et l'extrémité distale (62a) de tige sont exposés au-delà de l'extrémité distale (40a) de gaine.

3. Capteur selon la revendication 1, dans lequel la pluralité d'éléments (67) de capteur est exposée au niveau d'une ouverture (44) dans la gaine (40) adjacente à l'extrémité distale (40a) de gaine.

4. Capteur selon la revendication 1, dans lequel la pluralité d'éléments (67) de capteur est exposée au niveau d'ouvertures orthogonales (44) sur des côtés opposés de la gaine adjacentes à l'extrémité distale (40a) de gaine.

5. Capteur selon les revendications 1 à 4, dans lequel le raccord (46) inclut en outre un composant résilient (52) disposé à l'intérieur du raccord (46) contre le câble (50b) et un bouchon à pression (54) attaché de façon fixe au raccord (46) et dimensionné pour fournir une pression sur le composant résilient (52), faisant que le câble (50) et les plots (65) de connecteur restent en contact électrique intime.

6. Capteur selon les revendications 1 à 5, incluant en outre une électronique de conditionnement (70) couplée au câble (50), l'électronique de conditionnement (70) couplée en communication à un moniteur (4).

7. En combinaison, ensemble (30) de capteur intravasculaire et ensemble (20) de cathéter intraveineux, la combinaison comprenant :
un ensemble (20) de cathéter intraveineux comprenant un cathéter intraveineux (22) et une aiguille (24) d'insertion intraveineuse disposée amovible et coulissante à l'intérieur du cathéter intraveineux (20) ; et
un ensemble (30) de capteur intravasculaire comprenant une gaine (40), un raccord (46) connecté de façon étanche à une extrémité proximale (40b) de la gaine (40), et un capteur (60) disposé de façon étanche à l'intérieur de la gaine (40) dans laquelle le capteur (60) a une tige (62) de capteur avec une pluralité d'éléments (67) de capteur incluant une électrode de travail (67a) avec une matrice de réactif disposée sur celle-ci, la matrice de réactif comprenant une enzyme apte à catalyser une réaction impliquant un substrat pour l'enzyme et une électrode de référence (67b), la pluralité d'éléments (67) de capteur disposés adjacents à une extrémité distale (62a) de tige, une pluralité de plots (65) de connecteur disposés à une extrémité proximale (62a) de tige dans laquelle la pluralité de plots (65) de connecteur sont contenus à l'intérieur du raccord (46) et la pluralité d'éléments (67) de capteur sont exposés adjacents à une extrémité distale (40a) de gaine, et une pluralité d'éléments conducteurs (51) allongés dans laquelle chacun de la pluralité d'éléments conducteurs (51) couple électriquement un de la pluralité d'éléments (67) de capteur à un de la pluralité de plots (65) de connecteur, et un câble (50) électriquement couplé à la pluralité de plots (65) de connecteur ;
dans laquelle l'ensemble (30) de capteur intravasculaire est insérable de façon amovible et étanche dans le cathéter intraveineux (22) après suppression de l'aiguille d'insertion (24),
**caractérisée en ce que** :
l'extrémité proximale (62b) de tige de la tige (62) de capteur a une paire de pattes de contact (64) repliées orthogonalement vers l'extérieur dans des directions opposées depuis la tige de capteur de telle manière que les pattes de contact sont disposées sensiblement perpendiculaires à l'axe longitudinal de la tige de capteur, les pattes de contact ayant les plots (65) de connecteur exposés sur celles-ci, les plots de connecteur faisant tous face dans une direction ; et
les pattes de contact (64) sont logées contre une surface de référence (48) à l'intérieur du raccord (46) au niveau d'une base du raccord adjacente à une interface gaine-raccord.

8. Combinaison selon la revendication 7, dans laquelle la pluralité d'éléments (67) de capteur et l'extrémité distale (62a) de tige sont exposés au-delà de l'extrémité distale (40a) de gaine.

9. Combinaison selon la revendication 7, dans laquelle la pluralité d'éléments (67) de capteur est exposée au niveau d'une ouverture (44) dans la gaine adjacente à l'extrémité distale (40a) de gaine.

10. Combinaison selon la revendication 7, dans laquelle la pluralité d'éléments (67) de capteur est exposée au niveau d'ouvertures orthogonales (44) sur des côtés opposés de la gaine adjacentes à l'extrémité distale (40a) de gaine.

11. Combinaison selon les revendications 7 à 10, dans laquelle le raccord (46) inclut en outre un composant résilient (52) disposé à l'intérieur du raccord (46) contre le câble (50) et un bouchon à pression (54) attaché de façon fixe au raccord (46) et dimensionné pour fournir une pression sur le composant résilient (52), faisant que le câble (50) et les plots (65) de connecteur restent en contact électrique intime.
